# EUROPÄISCHE PATENTSCHRIFT

(11) **EP 1 256 358 B1**
(45) Veröffentlichungstag und Bekanntmachung des Hinweises auf die Patenterteilung: **29.09.2004**
(21) Anmeldenummer: 01111331.3
(22) Anmeldetag: 09.05.2001
(51) Int. Cl.: A61M 39/10, A61M 39/14

(54) **Infusionseinrichtung für die Verabreichung eines Therapeutikums**
Infusion set for drug delivery
Ensemble de perfusion pour l'administration d'un médicament

(43) Veröffentlichungstag der Anmeldung: 13.11.2002
(73) Patentinhaber: ARTA PLAST AB, 135 48 Tyresö (SE)
(72) Erfinder: Steg, Henning, 13550 Tyresö (SE)
(74) Vertreter: Niedmers, Ole, Dipl.-Phys.

(56) Entgegenhaltungen:
- US-A- 5 514 117
- US-A- 6 056 718
- US-A- 6 086 575
- US-A- 6 123 690

## Beschreibung

Die Erfindung betrifft eine Infusionseinrichtung für die Verabreichung eines Therapeutikums in das Körperinnere eines Menschen oder eines Tieres, umfassend wenigstens ein erstes Element, an dem eine Kanüle zum Einführen in das Körperinnere und zum Sitz im Körperinneren angeordnet ist, und ein zweites Element, das eine Nadel mit einem durchgehenden Hohlraum nach Art einer Injektionsnadel aufweist, die mit einem Schlauchelement für die Zufuhr des Therapeutikums verbunden ist, wobei das erste und das zweite Element über ein Rastelement lösbar derart miteinander verbindbar sind, daß das Therapeutikum vom Schlauchelement über die Nadel in die Kanüle gelangen kann.

Eine Injektionseinrichtung dieser Art ist bekannt (EP-B 0 688 232). Es sind viele Versuche unter Einschluß der vorgenannten bekannten Vorrichtung unternommen und in Form tatsächlich hergestellter Gegenstände dieser Art realisiert worden mit dem Ziel, die Handhabbarkeit derartiger Einrichtungen nicht nur für medizinisches Personal wie Ärzte, Schwestern und Pfleger zu verbessern, sondern auch für die Handhabung durch die Patienten bzw. Menschen selbst, die mit derartigen Infusionseinrichtungen im Zuge ihrer Selbstmedikation umgehen müssen. Es ist bekannt, daß beispielsweise an Zucker erkrankte Menschen sich regelmäßig selbst medikamentieren, sei es durch eigenständiges Spritzen des Medikaments mittels konventioneller Injektionsbestecke oder auch mittels der gattungsgemäßen Einrichtung.

Es sei aber vorangestellt, daß mittels der gattungsgemäßen Infusionseinrichtung grundsätzlich beliebige therapeutische Mittel in den Körper eines Menschen und auch eines Tieres eingeführt werden können, d.h. ihre Benutzung keineswegs auf die Verabreichung von Insulin beschränkt ist.

Insbesondere aber bei solchen Menschen, die in vorbestimmten Abständen regelmäßig ein therapeutisches Mittel verabreicht bekommen müssen, haben sich Infusionseinrichtungen der gattungsgemäßen Art als äußerst zweckmäßig herausgestellt, da somit fortwährende neue Einstiche durch die Haut und in die Venen bzw. Adern schlechthin vermieden werden, wodurch Schmerz und vielfach auch hämatombildende Einstiche von Injektionsnadeln auf ein absolut notwendiges Minimum begrenzt werden können. Bei der Anwendung der gattungsgemäßen Infusionseinrichtung kann in den allermeisten Fällen davon ausgegangen werden, daß die Menschen, die sich unter Zuhilfenahme der gattungsgemäßen Infusionseinrichtung selbst medikamentieren, konstitutionell geschwächt sind, und zwar einerseits durch die zu therapierende Krankheit und andererseits durch das Einführen der Kanüle in das Innere des eigenen Körpers. Wenn dann die körpereigene Schwäche noch von einem Widerwillen und einer damit verbundenen Unsicherheit des sich selbst medikamentierenden Menschen überlagert wird, kann es bei den bekannten Einrichtungen dieser Art unter Einschluß der o.g. gattungsgemäßen Vorrichtung zu Fehlhandhabungen bzw. Fehlbedienungen kommen. So ist bei der gattungsgemäßen Einrichtung nur dann eine Verbindung zwischen dem ersten und dem zweiten Element möglich, wenn das erste Element zum zweiten Element in einer, durch die dortige Konstruktion bedingt, eindeutigen Weise ausgerichtet ist. Eine Verdrehung beispielsweise des ersten und des zweiten Elements um 180° zueinander, d.h. anders, als die dortige Konstruktion es bedingt, ist nicht möglich. Auch in an sich richtiger Ausrichtung der beiden Elemente zueinander führt ein Verbindungsversuch nicht zu einem gewünschten Verbindungs- bzw. Verrastungserfolg, wenn das erste Element gegenüber dem zweiten Element auch nur geringfügig lateral verschoben ist, da neben den dortigen Rastelementen auch zwei in der gleichen Ebene angeordnete Führungselemente paßgenau auf die Führungsöffnungen im anderen Element ausgerichtet sein müssen, um ein verrastendes Einführen beider Elemente gegenseitig überhaupt herbeiführen zu können.

Ein kranker Mensch, insbesondere wenn dieser fortgeschrittenen Alters ist, ist in den allermeisten Fällen nicht in der Lage, die richtige Ausrichtung sowohl in Rotationsrichtung als auch in lateraler Richtung der beiden Elemente zueinander zu bewerkstelligen. Hinzu kommt noch, daß der Verbindungsvorgang zweier Elemente, durch den die Infusionseinrichtung benutzenden Menschen regelmäßig mit einer Hand durchgeführt werden muß, da die Kanüle der gattungsgemäßen Infusionseinrichtung beispielsweise in die Vene eines Arms eingeführt wird, so daß die Hand dieses Arms für den Einführvorgang nicht zur Verfügung steht.

Ein zusätzlicher konstruktiver Aspekt der eingangs genannten Infusionseinrichtung soll hier ebenfalls nicht unerwähnt bleiben. Aufgrund der erwähnten Ausgestaltung mit zwei zungenförmigen Rastelementen und zwei stiftförmigen Führungselementen in einem Element der Einrichtung und aufgrund von zwei Rastelementführungen bzw. Verrastungsöffnungen und zwei Öffnungen für die Aufnahme der Führungsstifte, die alle paßgenau zueinander fluchten müssen, sind allerhöchste Ansprüche an die Präzision der Werkzeuge, mit denen derartige Einrichtungen hergestellt werden, gestellt, was dazu führt, daß die Einrichtung selbst auch nur auf sehr aufwendige und kostspielige Weise hergestellt werden kann. Zum anderen muß aufgrund dieser Umstände auch die Herstellung der bekannten Infusionseinrichtung mit den Werkzeugen derart präzise sein, daß die oben erwähnte paßgenaue Ausrichtung der Verrastungszungen mit den Öffnungen im anderen Element sowie die Ausrichtung der beiden Führungsstifte in den Öffnungen des anderen Elements immer gewährleistet ist. Dieses impliziert natürlich auch, daß die Nadel im zweiten Element immer paßgenau im verrasteten Zustand beider Elemente in die im einen Element angeordnete Öffnung der Kanüle ragt bzw. zu dieser axial ausgerichtet ist. Somit ist nicht nur das Herstellungswerkzeug für die gattungsgemäße Infusionseinrichtung bei der bekannten Einrichtung sehr kostspielig herstellbar, sondern auch der eigentliche Herstellungsvorgang kostspielig, um eine fortwährende präzise Ausrichtung aller ineinandergreifender Teile zu gewährleisten.

Es ist somit Aufgabe der vorliegenden Erfindung, eine Infusionseinrichtung der eingangs genannten Art zu schaffen, mit der es insbesondere sowohl dem ungeübten Benutzer als auch dem geschwächten Benutzer möglich ist, auf einfache Weise zunächst die Kanüle in das Körperinnere einzuführen und auf einfache Weise auch die Verbindung mit dem in das Körperinnere einzuführenden Therapeutikum mittels der beiden Elemente herzustellen, ohne daß eine aufwendige und in Rotationsrichtung der beiden Elemente zueinander richtige Ausrichtung eingehalten werden muß, wobei ein einfaches Verriegeln und Entriegeln der beiden Elemente miteinander bzw. voneinander möglich sein soll und fortwährend eine hochpräzise Ausrichtung der Nadel des einen Elements zur Eintrittsöffnung der Kanüle im anderen Element erreicht wird, und wobei die Infusionseinrichtung einfach hergestellt werden kann, so daß sie sich auch zur massenhaften Weitergabe und einem massenhaften Einsatz aufgrund verhältnismäßig gering angestrebter Herstellungskosten auf für die für die Herstellung nötigen Werkzeuge eignet.

Gelöst wird die Aufgabe gemäß der Erfindung dadurch, daß das Rastelement durch eine im wesentlichen kreisringförmig umlaufende Nut des im wesentlichen axialsymmetrisch zur Nadel ausgebildeten zweiten Elements und durch ein im Verbindungszustand im wesentlichen orthogonal zum zweiten Element im ersten Element geführtes hin- und herverschiebbares Klauenelement, das im Verbindungszustand beider Elemente in die Nut eingreifend beide Elemente miteinander verriegelt hält, ausgebildet wird.

Der Vorteil der erfindungsgemäßen Lösung besteht im wesentlichen darin, daß, wie angestrebt, ein einfaches Einführen des zweiten Elements in das erste Element möglich ist, ohne daß eine rotationsrichtige bzw. in lateraler Richtung der beiden Elemente zueinander richtige Ausrichtung gewählt werden muß. Das zweite Element braucht im eigentlichen Wortsinne lediglich in eine entsprechende einzige Öffnung im ersten Element hineingesteckt zu werden, wie man dieses von Koaxialsteckern im Bereich der Elektrotechnik bzw. Elektronik kennt. Eine rotationsrichtige Ausrichtung ist nicht erforderlich. Sind beide Elemente nicht axial zueinander richtig ausgerichtet, kann auch keine Fehlbedienung erfolgen, da das zweite Element dann überhaupt nicht, auch nicht mit irgendwelchen Stift- und Rastelementen, die bekannte Einrichtung weist davon vier vom zweiten Element vorstehende auf, fehlerhaft eingeführt werden kann. Das Rastelement selbst braucht beim Zusammenführen und Verriegeln beider Elemente überhaupt nicht betätigt zu werden, wohingegen es zum Entriegeln lediglich z.B. mit Daumen und Zeigefinger betätigt zu werden braucht. Ein weiterer Vorteil der erfindungemäßen Lösung ist auch darin zu sehen, daß aufgrund der verhältnismäßig einfachen aber in bezug auf Verriegelung und Entriegelung und Ausrichtung der Nadel zur Öffnung der Kanüle einfachen aber dennoch sehr - in bezug auf die Führung der beiden Elemente - präzisen Konstruktion die Werkzeugkosten für die Herstellung einer derartigen Infusionseinrichtung und für die Herstellung der Infusionseinrichtung als solcher drastisch reduziert werden konnten, so daß auch das angestrebte Ziel einer massenhaften Bereitstellbarkeit zu geringen Kosten erreicht wird.

Um die Einführbarkeit des zweiten Elements in das für das zweite Element im ersten Element ausgebildete Eintrittsloch noch weiter zu erleichtern, weist das zweite Element einen im wesentlichen kreisförmigen Querschnitt auf, bei dem die Kreisachse bzw. der Kreismittelpunkt mit der Achse der Nadel zusammenfällt.

Eine weitere Maßnahme zur Erleichterung der Einführung des zweiten Elements in das Eintrittsloch im ersten Element wird dadurch erreicht, daß der in das erste Element hineinzuführende Abschnitt des zweiten Elements an seinem freien Ende eine umlaufende Abschrägung aufweist. Dadurch erfolgt auch bei nicht präziser lateraler Ausrichtung der beiden Elemente zueinander eine leichte Einführbarkeit des zweiten Elements in das Eintrittsloch, da dadurch selbständig eine korrektere axiale Ausrichtung bei weiterem Hineinschieben des zweiten Elements gewissermaßen geführt erfolgt. Die umlaufende Abschrägung hat aber auch noch die Funktion, daß dadurch, daß das Klauenelement durch die Schrägung bei axialem Einführen des zweiten Elements in das Eintrittsloch auseinandergedrückt wird, die Spreizung der Klauenschenkel erfolgt, d.h. die Abschrägung wirkt als schiefe Ebene für den Spreizungsvorgang der klauenschenkel. Auch das Eintrittsloch des ersten Elements kann sich in seinem äußeren Randbereich beispielsweise abgeschrägt erweiternd ausgebildet sein, um so beispielsweise eine trichterförmig ausgebildete Einführöffnung zu bilden.

Das Klauenelement wird vorzugsweise durch zwei gesonderte Klauenschenkel gebildet, die im wesentlichen parallel zueinander geführt aufeinander zu- bzw. voneinander wegbewegbar sind. Im Zuge der Aufeinanderzubewegung wird das Eintrittsloch des ersten Elementes entriegelt, so daß das zweite Element in das Eintrittsloch bis zu einer vorbestimmten Endstellung hineingeschoben werden kann, wohingegen im Zuge der Voneinanderwegbewegung die Klauenschenkel des Klauenelements das zweite Element im ersten Element lösbar verriegelbar festgehalten wird.

Vorteilhafterweise weisen die Klauenschenkel einen kreisabschnittförmigen Bereich mit einem Radius entsprechend dem Radius der Nut des zweiten Elements auf, mit dem er in die Nut verriegelnd eingreift. Der kreisabschnittförmige Bereich ist in diesem Falle vorzugsweise annähernd viertelkreisförmig, wobei beide viertelkreisförmigen Bereiche der beiden Klauenschenkel zusammen einen halbkreisförmigen, kreisabschnittförmigen Bereich bilden, mit dem in Verriegelungsstellung das zweite Element sicher gehalten werden kann.

Um auf einfache Weise zu bewirken, daß die beiden das Klauenelement bildenden Klauenschenkel bei der Aufeinanderzubewegung das Eintrittsloch des ersten Elements für das zweite Element freigeben bzw. bei eingestecktem zweiten Element in eine Entriegelungsstellung, falls gewünscht, zu gehen, so daß das zweite Element aus dem ersten Element herausgezogen werden kann, weist jeder Klauenschenkel einen in Form einer schiefen Ebene ausgebildeten Bereich auf, wobei in der als Führungsschlitz ausgebildeten Führung im ersten Element ebenfalls als schiefe Ebene ausgebildete, gegensinnig geneigt für jeweils einen Klauenschenkel ausgebildete Bereiche vorgesehen sind, wobei zum Entriegeln der beiden Elemente voneinander die als schiefe Ebene ausgebildeten Bereiche der jeweiligen Klauenschenkel an den jeweils zugeordneten, als schiefe Ebene ausgebildeten Bereichen des ersten Elements aufgleiten und dabei die Klauenschenkel derart elastisch verformt werden, daß sie außer Eingriff mit der Nut kommen. Die sich bei der elastischen Verformung aufbauende Rückstellkraft ist so groß, daß dann, wenn die Klauenschenkel bzw. das Klauenelement losgelassen wird, selbständig wieder die Verriegelungsstellung des Klauenelements eingenommen wird.

Gemäß einer weiteren vorteilhaften Ausgestaltung der Infusionseinrichtung ist die Führung für das Klauenelement bzw. die Klauenschenkel im wesentlichen orthogonal zur Achse des im wesentlichen im Querschnitt kreisförmigen Eintrittslochs für das zweite Element ausgebildet.

Die Infusionseinrichtung ist auch zum Betrieb durch Rechtshänder oder Linkshänder bzw. Rechtshandbetrieb und Linkshandbetrieb schlechthin geeignet, da die das Klauenelement bildenden beiden Klauenschenkel aufgrund ihres im wesentlichen identischen Aufbaus und ihrer Anordnung und ihrer Verschiebbarkeit symmetrisch zur Achse, zu der axialsymmetrisch auch die Kanüle angeordnet ist und auch der gleiche Bewegegungsablauf der Klauenschenkel beim Verriegeln und beim Entriegeln des zweiten Elements erreicht wird, unabhängig davon, ob die beiden Klauenschenkel mit den Fingern der rechten Hand oder den Fingern der linken Hand betätigt werden.

Um auf einfache Weise sicherzustellen, daß die Bewegungsbahn der Klauenschenkel innerhalb des Führungsschlitzes im ersten Element begrenzt wird, weisen die Klauenschenkel vorzugsweise eine Rastnase auf, mit der sie hinter Rastvorsprünge, die im Bereich der Führung bzw. des Führungsschlitzes im ersten Element ausgebildet sind, nach Art einer Klippverbindung rastend eingreifen. Es sind somit keine weiteren Maßnahmen erforderlich, um die Klauenschenkel in dem Führungsschlitz zu halten, nachdem die Klauenschenkel bei der Montage einmal mit ihren Rastnasen hinter den Rastvorsprüngen im Führungsschlitz zu liegen gekommen sind.

Das freie Element des Rastelementes kann vorzugsweise mit einer Handhabe versehen sein, die beispielsweise in Form einer Verbreiterung des aus dem Körper des ersten Elements herausstehenden Klauenelements ausgebildet sein kann. Die Handhabe erleichtert es dem Benutzer der Einrichtung, das Klauenelement bequemer zu betätigen, wobei es von Vorteil sein kann, daß dabei ebenfalls die Handhabe zusätzlich noch im ersten Element geführt aufgenommen wird, wodurch insgesamt die Führung des Klauenelements im ersten Element noch verbessert werden kann.

Um ein Abrutschen des Fingers von der Handhabe bzw. dem herausstehenden Ende des Klauenelements aus dem ersten Element auszuschließen, weist die Handhabe vorteilhafterweise an ihrer Betätigungsseite ein griffverbesserndes Mittel auf, beispielsweise in Form von Rillungen, Riefungen, eines stumpfen Belags oder dgl.

Aus dem gleichen Grunde ist es vorteilhaft, die der Handhabe gegenüberliegende Seite des ersten Elements wenigstens teilweise mit einem griffverbessernden Mittel zu versehen, so daß dann, wenn beispielsweise zwischen Daumen und Finger die Handhabe bzw. das Klauenelement und das erste Element ergriffen wird, ein sicherer Sitz bzw. ein sicheres Ergreifen mit beispielsweise Daumen und Finger gewährleistet ist.

Gemäß einer noch anderen vorteilhaften Ausgestaltung der Erfindung weist das zweite Element an seinem freien Ende eine im wesentlichen axiale Vertiefung auf, wobei vorzugsweise die Nadel dabei mit ihrem spitzen Bereich in die Vertiefung derart hineinsteht, daß sie über das freie Ende des zweiten Elements nicht hinausragt. So ist fortwährend sichergestellt, daß beim Einführen des zweiten Elements in das Eintrittsloch des ersten Elements keine Berührung der Nadel durch den Benutzer erfolgen kann, so daß eine Kontamination mit Schmutz und Mikroorganismen immer vermieden wird und auch keine mechanische Beschädigung dieses Bereiches der Nadel geschehen kann, der in die axiale Vertiefung hineinsteht, so daß eine fortwährende präzise axiale Ausrichtung zum Öffnungsbereich der Kanüle im ersten Element gewährleistet ist.

Um eine gute mechanische Stabilität zwischen beiden Elementen im miteinander verriegelten Zustand zu erreichen, was sehr förderlich auch für die fortwährende Ausrichtung des Spitzenbereichs der Nadel zum Öffnungsbereich der Kanüle ist, ist die Infusionseinrichtung dazu vorzugsweise derart ausgestaltet, daß im miteinander verbundenen und verriegelten Zustand beider Elemente der elementseitige Öffnungsbereich der Kanüle wenigstens teilweise in die Vertiefung hineinragt. So ist gewährleistet, selbst wenn der Werkstoff beider Elemente in gewissen Bereichen elastisch verformbar ist bzw. aufgrund äußerer Einflüsse auch geringfügig verformt wird, daß fortwährend die zwingend notwendige axiale Ausrichtung zwischen der Spitze der Nadel und dem Öffnungsbereich der Kanüle erreicht ist.

Es gibt verschiedene Möglichkeiten im verbundenen Zustand beider Elemente die Spitze der Nadel gegen die Umgebung dichtend in der Öffnung der Kanüle im ersten Element abzudichten. So ist es beispielsweise möglich, den Innendurchmesser der Kanülenöffnung auf den Außendurchmesser der Nadelspitze so abzustimmen, daß die Spitze der Nadel im verbundenen Zustand im Paßsitz in der Öffnung bzw. im Öffnungsbereich der Kanüle zu sitzen kommt. Diese Art der Ausgestaltung erfordert aber eine außerordentlich hohe Präzision sowohl der Herstellung des ersten Elements als auch des zweiten Elements, da das Eingehen eines Paßsitzes nur dann möglich ist, wenn die Achse der rohrförmig ausgebildeten Kanüle präzise zur Achse der rohrförmig ausgebildeten Nadel ausgerichtet ist. Eine andere vorteilhafte Ausgestaltung der Einrichtung zur Sicherstellung des nach außen dichten Bereiches zwischen Öffnung der Kanüle und der Öffnung in der Nadelspitze wird dadurch erreicht, daß der Öffnungsbereich mit einem diesen abdeckenden Verschlußelement versehen ist, das beim Einführen der Nadel in den Öffnungsbereich, d.h. beim Einführen des zweiten Elements in das Eintrittsloch des ersten Elements, durchstoßen wird bzw. ist, wenn der bestimmungsgemäße Sitz beider Elemente zueinander erreicht ist, bei dem die gegenseitige Verriegelungsstellung eingenommen wird.

Um aber für diesen Fall dafür zu sorgen, daß nach dem Entriegeln der beiden Elemente und dem Herausziehen des zweiten Elements aus dem Eintrittsloch Schmutz und Mikroorganismen in die somit freiliegende Öffnung der Kanüle nicht eintreten kann bzw. können, wird das Verschlußelement vorzugsweise aus einem elastomeren und/oder plastomeren Werkstoff ausgebildet, wodurch erreicht werden kann, daß einerseits die Nadel das derart ausgebildete Verschlußelement leicht durchdringen kann und andererseits nach dem Herausziehen der Nadel aufgrund der elastischen bzw. plastischen Eigenschaften des Werkstoffs des Verschlußelementes die durch die Nadel geschaffene Durchtrittsöffnung wieder verschlossen wird.

Die wesentlichen Elemente der Infusionseinrichtung, d.h. wenigstens das erste und das zweite Element sowie das Rastelement, können aus Kunststoff hergestellt werden, wobei dafür beliebige Kunststoffe geeignet sind, die human- und tiermedizinisch unbedenklich bzw. neutral sind. Vorzugsweise sind diese Werkstoffe spritzfähige Werkstoffe, so daß die Infusionseinrichtung vorteilhafterweise mittels Kunststoffspritzmaschinen hergestellt werden kann. Als Werkstoff eignet sich beispielsweise Polycarbonat gut.

Da sich das Klauenelement bzw. die das Klauenelement bildenden beiden Klauenschenkel bestimmungsgemäß bei Einnahme der Entriegelungsstellung bestimmungsgemäß beträchtlich elastisch verformen müssen, ist es ganz besonders vorteilhaft, das Klauenelement aus Polyacetat POM herzustellen, da dieser Werkstoff bei großer Stabilität sehr gute elastische Verformungseigenschaften aufweist.

Die Kanüle kann prinzipiell bei Fertigung des ersten Elements im Zuge des Spritzvorganges im ersten Element fest angeordnet bzw. mit diesem fest verbunden werden. Es hat sich jedoch als äußerst vorteilhaft herausgestellt, die Kanüle an ihrem im ersten Element zu liegen kommenden Kanülenende mit einem Hülsenelement zu umgeben, über das die Kanüle in einer am Ende des Eintrittlochs für das zweite Element sacklochartig ausgebildeten Öffnung im ersten Element aufgenommen wird. So kann einerseits das erste Element unabhängig von der Kanüle hergestellt werden und es besteht die Möglichkeit, die gesondert gefertigte Kanüle über das Hülsenelement dann über Einsetzen in die sacklochartige Öffnung mit dem ersten Element zu verbinden. Dieses ist beispielsweise auch für Austausch- und/oder Reinigungszwecke der Kanüle von sehr großem Vorteil.

Um keine weiteren Befestigungsmittel vorsehen zu müssen, um die Kanüle im ersten Element sicher aufzunehmen, ist es vorteilhaft, daß die Öffnung einen geeignet ausgebildeten Sitz, vorzugsweise einen Preßsitz, für das Hülsenelement bildet.

Ebenfalls vorteilhaft ist es, das Verschlußelement, durch das die Nadel bei eingesetztem zweiten Element hindurchgeht bzw. das eine staubdichte bzw. eine mikroorganismusdichte oder gegen sonstige Verunreinigungen dichte Barriere bildet, wenn das zweite Element mit seiner Nadel nicht durch das Verschlußelement hindurchsteht, im Hülsenelement selbst anzuordnen. Somit kann auch durch einfaches Austauschen der Kanüle gleichzeitig auch das Verschlußelement bei Beschädigungen oder sonstigen Beeinträchtigungen ausgetauscht und im Zuge des Einsatzes einer neuen Kanüle ersetzt werden.

Schließlich kann im Hülsenelement in Richtung des austrittseitigen Endes des Therapeutikums aus der Kanüle vor dem Verschlußelement ein Verbindungselement angeordnet sein, über das die Kanüle mit dem Hülsenelement verbunden ist. Somit kann im Hülsenelement neben dem Verschlußelement das Verbindungselement angeordnet sein, und es wird in der Hülse davor ebenfalls die Kanüle über das Verbindungselement aufgenommen.

Um das Einführen der Kanüle in das Körperinnere zu erleichtern, ist das zweite Element in modifizierter Ausführungsform mit einer derart langen Nadel versehen, so daß diese im verrasteten Zustand des ersten und zweiten Elements aus der zum Austritt des zu verabreichenden Therapeutikums normalerweise bestimmten Spitze der Kanüle heraussteht. Ist die Kanüle mit der Nadel dann in das Körperinnere eingeführt, wird das derart modifizierte zweite Element herausgezogen und das zweite Element in der eingangs beschriebenen Grundausgestaltung mit der hohlraumförmigen Nadel für die Zuführung eines Therapeutikums wird auf vorbeschriebene Weise in das erste Element hineingesteckt und in Endstellung auf vorbeschriebene Weise verriegelt.

Die Infusionseinrichtung kann in Form ihrer einzelnen, voneinander getrennten Elemente sterilisiert werden oder aber auch im zusammengefügten Zustand, und zwar mit ionisierenden Strahlen, beispielsweise β-Strahlen. Es ist aber auch möglich, eine Sterilisierung mittels eines oxidierenden Gases, beispielsweise Ethylengas, vorzunehmen. Um sicherzustellen, daß auch der Innenbereich des ersten Elements bei zusammengefügtem Zustand der beiden Elemente, bei dem das erste Element mit dem modifizierten zweiten Element verbunden ist, vom oxidierenden Gas erreicht wird, ist ebenfalls die lange Nadel nach Art einer Infusionsnadel mit einem axial durch die Nadel hindurchgehenden Hohlraum versehen, wobei eine vom Äußeren der Nadel aus in den Hohlraum hineinführende Öffnung vorgesehen ist, die im zusammengeführten Zustand des ersten und des zweiten Elements im Bereich der elementseitigen Öffnung der Kanüle bzw. der dortigen Kanülenöffnung vorgesehen ist. So kann von der Nadelspitze aus Gas in den inneren elementseitigen Hohlraum gelangen, die Umgebung dort sterilisieren und dann zwischen dem zwischen Kanüle und Nadel gebildeten Hohlraum wieder nach außen geführt werden.

Die Erfindung wird nun unter Bezugnahme auf die nachfolgenden schematischen Zeichnungen anhand eines Ausführungsbeispieles eingehend beschrieben. Darin zeigen:
- Fig. 1: in der Draufsicht die Einrichtung aus erstem und zweitem Element in verriegeltem Zustand,
- Fig. 2: einen Schnitt der Linie A - B von Fig. 1,
- Fig. 3: eine Ansicht auf das erste Element von unten,
- Fig. 4a: eine Seitenansicht des linken Klauenschenkels im Schnitt,
- Fig. 4b: eine Draufsicht auf den linken Klauenschenkel gemäß Fig. 4a,
- Fig. 5a: eine Seitenansicht des rechten Klauenschenkels im Schnitt,
- Fig. 5b: eine Draufsicht auf den rechten Klauenschenkel gemäß Fig. 5a,
- Fig. 6: eine Seitenansicht gemäß Fig. 1 in geschnittenem Zustand entlang der Linie C - D von Fig. 1,
- Fig. 7: eine Seitenansicht gemäß Fig. 1 in geschnittenem Zustand entlang der Linie C - D von Fig. 1, wobei das erste Element mit einem modifizierten zweiten Element verbunden ist, bei dem die Nadelspitze des zweiten Elements aus der Kanüle heraussteht,
- Fig. 8: die Kanüle mit Hülsenelement sowie Verschlußelement und Verbindungselement im Schnitt,
- Fig. 9: im Schnitt das zweite Element der Einrichtung und
- Fig. 10: im Schnitt das erste Element der Einrichtung, wobei durch die gestrichelte Verbindungslinie mit Fig. 9 die Verbindbarkeit zwischen erstem und zweitem Element angedeutet ist.

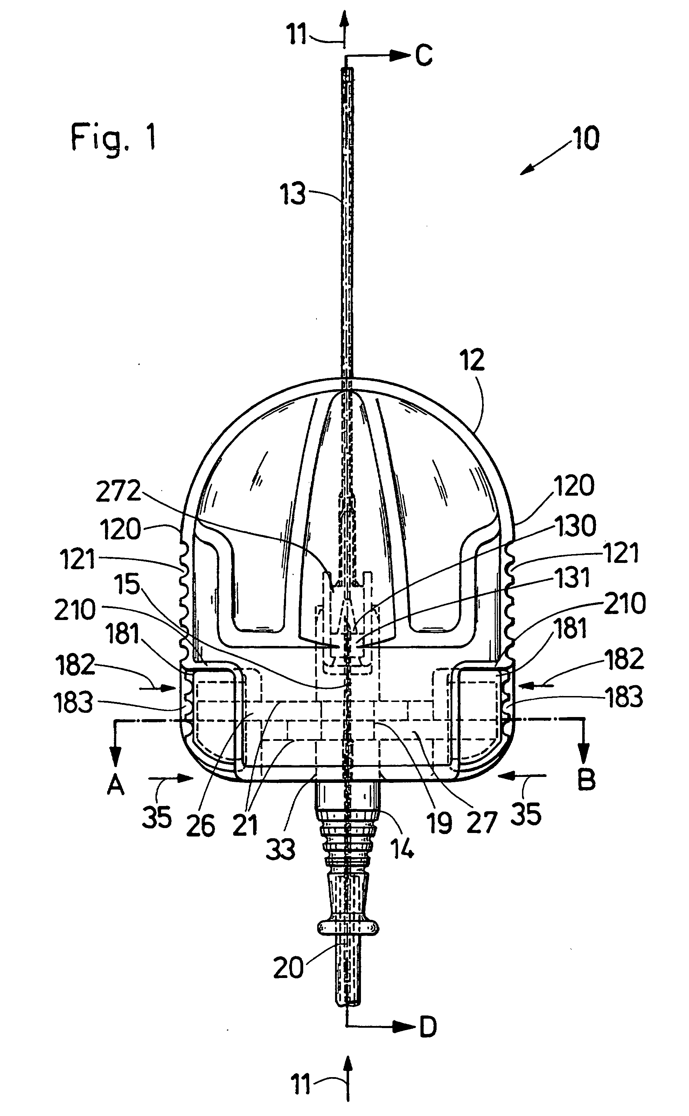

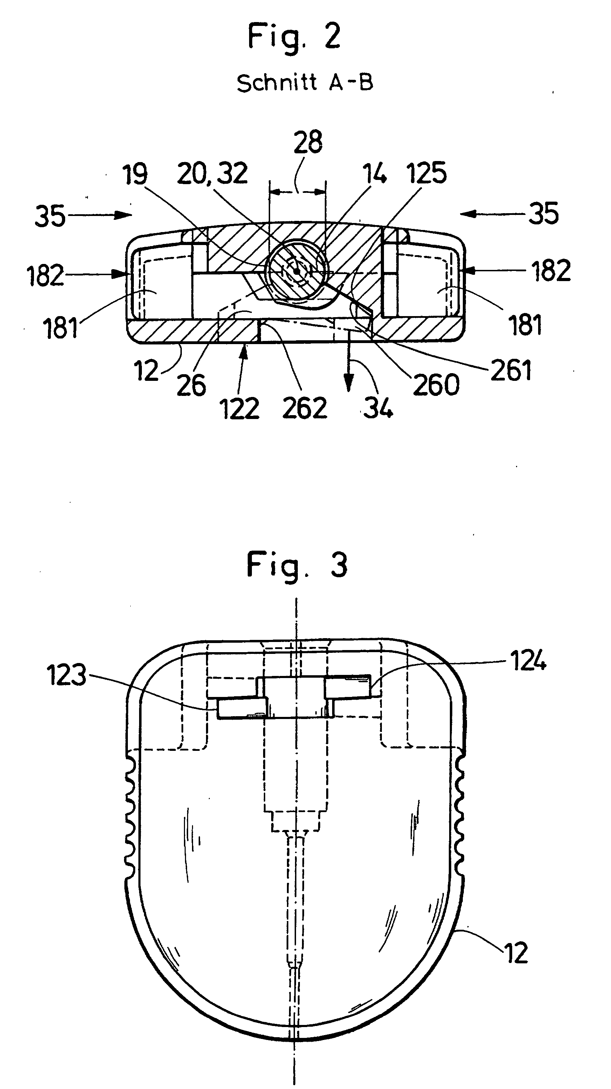

Es wird zunächst Bezug genommen auf die Darstellung von Fig. 1, aus der die Infusionseinrichtung 10 in ihrer Gesamtheit in der Draufsicht ersichtlich ist. Die Infusionseinrichtung 10 umfaßt wenigstens ein erstes Element 12, an dem eine Kanüle 13 zum Einführen in das Körperinnere eines Benutzers (Mensch, Tier) (nicht dargestellt) angeordnet ist, sowie ein zweites Element 14. Das zweite Element 14 weist eine Nadel 15 auf, die im wesentlichen in der Längsachse 20 des zweiten Elements 14 angeordnet ist. Die Nadel 15 ist auf an sich bekannte Weise mit einem axial hindurchgehenden Hohlraum versehen, d.h. nach Art einer bekannten Injektionsnadel ausgebildet. Die Nadel 15 ist im zweiten Element 14 mit einem Schlauchelement 17 für die Zufuhr eines Therapeutikums verbunden, wobei dessen Zufuhr in Fig. 1 durch den Pfeil 11 angedeutet ist. Im Zustand der Verriegelung des ersten Elements 12 mit dem zweiten Element 14, wie es in Fig. 1 dargestellt ist, wird das Therapeutikum 11 über das Schlauchelement 17 über die Nadel 15 in die Kanüle 13 überführt und gelangt von dort, wenn die Kanüle 13 in das Körperinnere eines Menschen oder eines Tieres eingeführt ist, in den Organismus. Um in diesem Verriegelungszustand einer fortwährend sicheren Sitz des zweiten Elements 14 im ersten Element 12 sicherzustellen, ist ein Rastelement vorgesehen, das in Form eines Klauenelements 22, vgl. Fig. 2, ausgebildet ist.

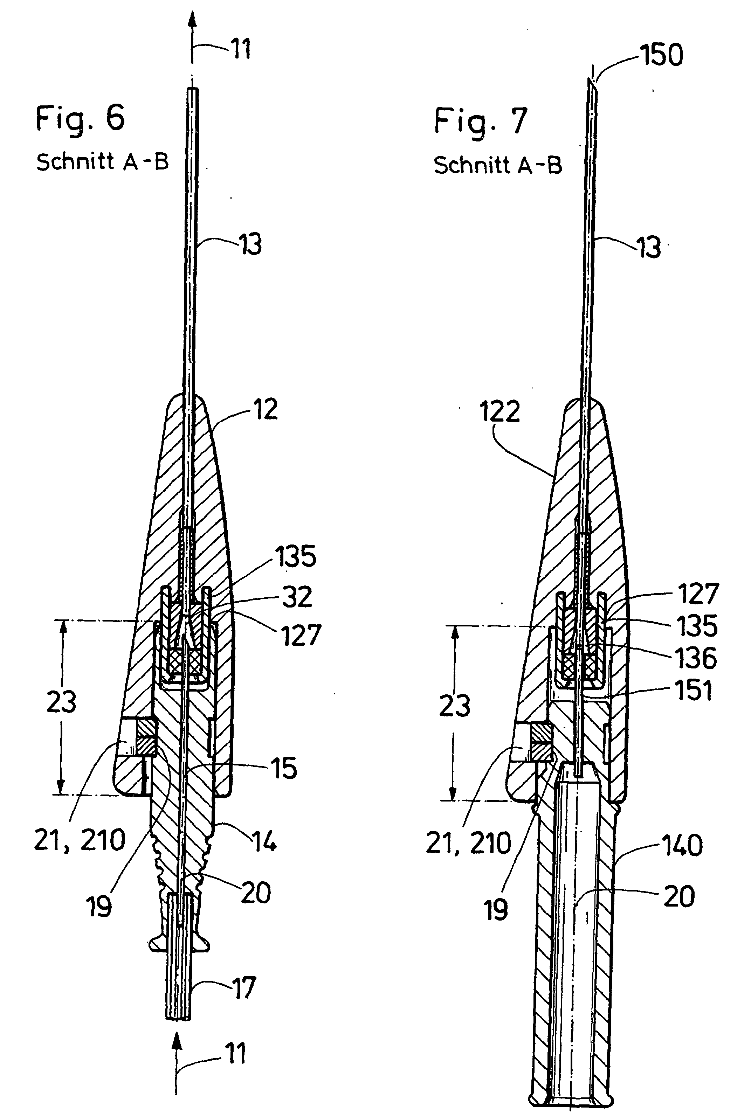

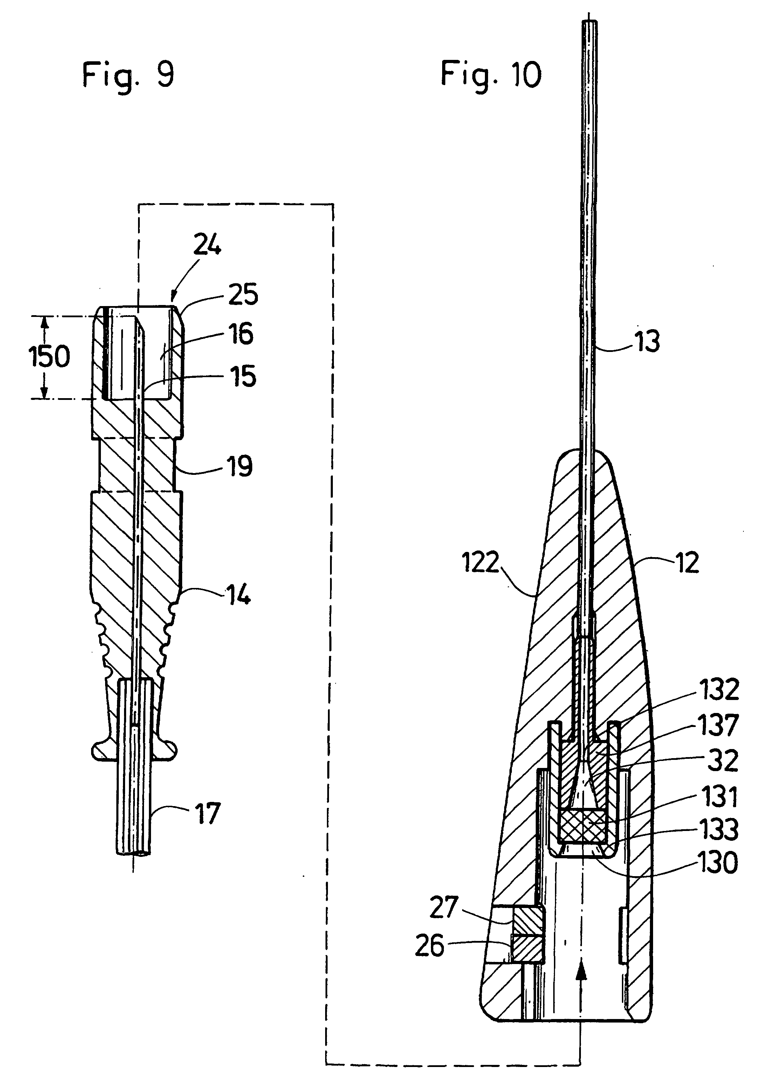

Das Rastelement umfaßt neben dem Klauenelement 22 eine am im wesentlichen rotationssymmetrisch und im Querschnitt kreisförmig ausgebildeten zweiten Element 14 ausgebildete Nut 19, vgl. insbesondere die Fig. 2 und 9 und angedeutet in Fig. 1. Die Nut 19 umläuft das zweite Element 14 im wesentlichen kreisförmig. Die Nut 19 ist im Bereich des Einführabschnittes 23, vgl. Fig. 6 und 7, ausgebildet, d.h. in dem Abschnitt des zweiten Elements 14, der im Verriegelungszustand im Eintrittsloch 33 des ersten Elements 12 liegt.

Das Teil des Rastelements, d.h. das Klauenelement 22, das im ersten Element 12 ausgebildet ist, wird durch eine im wesentlichen orthogonal zur Achse 32 des Eintrittslochs 33 ausgebildete Führung 21 geführt, d.h. die Führung 21 kann lediglich eine einfache schlitzförmige Vertiefung sein, vgl. Fig. 1, in der das Klauenelement 22 hin- und herverschiebbar ist, vgl. die Fig. 1 und 2.

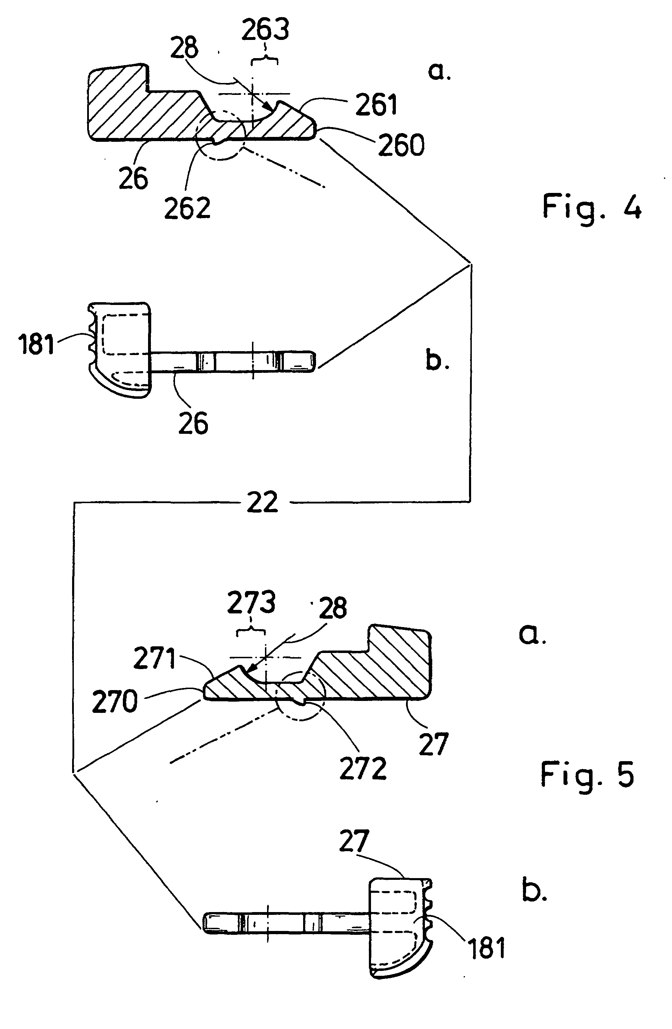

Es wird weiterhin Bezug genommen auf die Fig. 4a, 4b sowie 5a, 5b, die das Klauenelement 22 zeigen, das den Teil des Rastelements bildet, der im ersten Element 12 angeordnet ist. Das Klauenelement 22 wird durch zwei gesonderte Klauenschenkel 26, 27 gebildet. Die Klauenschenkel 26, 27 sind grundsätzlich identisch ausgebildet, wobei lediglich die mit den Klauenschenkeln 26, 27 jeweils verbundene Handhabe 181, die mit den Klauenschenkeln 26, 27 integral ausgebildet sein kann, bei der hier dargestellten Ausgestaltung eine in bezug auf die Darstellung von Fig. 1 linke Version (Klauenschenkel 26) und eine rechte Version (Klauenschenkel 27) bilden. Die Klauenschenkel 26, 27 weisen einen kreisabschnittförmigen Bereich 263, 273 auf. Dort ist ein kreisabschnittförmiger Bereich 263, 273 vorgesehen. Der Radius 28 dieses kreisabschnittförmigen Bereiches 263, 273 entspricht dem Radius 28, der Nut 19 des zweiten Elements. Der kreisabschnittförmige Bereich 263, 273 ist hier annähernd viertelkreisförmig ausgebildet. An den Klauenschenkeln 26, 27 sind in Form einer schiefen Ebene ausgebildete Bereiche 261, 271 vorgesehen, die sich zu den freien Enden 260, 270 hin verjüngen bzw. in bezug auf die schiefe Ebene 261, 271 abfallen.

Im ersten Element 12, vergleiche Fig. 2, sind ebenfalls als schiefe Ebene ausgebildete Bereiche 125, 126 vorgesehen, und zwar in der dort als Führungsschlitz 210 ausgebildeten Führung 21, vergleiche Fig. 1, 6 und 7.

Werden die Handhaben 181 der beiden Klauenschenkel 26, 27 in Richtung der Pfeile 35, vergleiche Fig. 2, verschoben, d.h. gegensinnig, um ein Entriegeln der beiden Elemente 12, 14 voneinander zu ermöglichen, gleiten die als schiefe Ebene ausgebildeten Bereiche 261, 271 der Klauenschenkel 26, 27 auf die ebenfalls als schiefe Ebene ausgebildeten Bereiche 125, 126 in der Führung 21 des ersten Elements 12 auf. In Folge dieses Aufgleitens werden die Klauenschenkel 26, 27 aufgrund geeigneter Werkstoffwahl elastisch verformt, was in Fig. 2 für den Klauenschenkel 26 in Form einer gestrichelten Linie angedeutet ist, und zwar in Richtung des Pfeiles 34. Gleiches gilt auch für den Klauenschenkel 27 (in Fig. 2 nicht dargestellt). Bei weiterer Bewegung der beiden Klauenschenkel 26, 27 in Richtung der Pfeile 35 kommen die Klauenschenkel 26, 27 außer Eingriff mit der Nut 19 des zweiten Elements 14, so daß das zweite Element 14 freikommt und aus dem ersten Element 12 herausgezogen werden kann.

Die Klauenschenkel 26, 27 werden in der Führung 21, 210 parallel zueinander geführt und sind in dieser in Richtung des Pfeiles 35 aufeinander zu bewegbar und in entgegengesetzter Richtung zum Pfeil 35 bewegbar, wobei die Bewegung entgegen der Richtung der Pfeile 25 aufgrund der elastischen Gegenkraft der Klauenschenkel 26, 27 selbsttätig erfolgen kann. Es brauchen also für die Bewegung der Klauenschenkel 26, 27 entgegen der Richtung der Pfeile 35 die jeweiligen Handhaben 181 lediglich losgelassen zu werden. Infolgedessen wird selbständig der Verriegelungszustand der Klauenschenkel 26, 27 bzw. des Klauenelements 22 eingenommen. Gute elastische Verformungseigenschaften bei dennoch großer Haltbarkeit weist beispielsweise Polyacetat POM auf, aus dem die Klauenschenkel 26, 27 vorzugsweise hergestellt sind.

Die Klauenschenkel 26, 27 weisen auch eine Rastnase 262, 272, vergleiche die Fig. 4a, 5a sowie 2, auf. Mit den Rastnasen 262, 272 greifen sie hinter Rastvorsprünge 123, 124, die im Bereich der Führung 21 im ersten Element ausgebildet sind, vergleiche Fig. 3. Die Rastnasen 262, 272 bilden zusammen mit den Rastvorsprüngen 123, 124 eine Klippverbindung, die eingegangen wird, wenn die Klauenschenkel 26, 27 in die Führung 21, 210 bei der Montage mit dem ersten Element 12 eingeführt werden. Die Rastnasen 262, 272 bilden neben einer Befestigung der Klauenschenkel 26, 27 im ersten Element 12 auch eine Bewegungsbegrenzung der Klauenschenkel 26, 27 entgegen der Richtung der Pfeile 35, vergleiche Fig. 2.

Umgekehrt gibt es für das Einführen des zweiten Elements 14 in das Eintrittsloch 33 des ersten Elements 12 zum Erreichen eines sicheren verriegelten Sitzes, bei dem die Nadel 15 im Öffnungsbereich 130 der Kanüle 13 sitzt, vgl. Fig. 1 und 6, zwei Möglichkeiten.

Entweder wird das zweite Element 14 einfach in das Eintrittsloch 33 hineingeführt, bis es mit seinem freien Ende 24 an die Klauenschenkel 26, 27, vgl. Fig. 2, stößt. Das freie Ende 24 ist derart gestaltet, daß es eine umlaufende Abschrägung 25 aufweist. Die Abschrägung 25 ist derart gewählt, daß sie die Klauenschenkel 26 an den Schenkelinnenseiten 263, 273 geringfügig ergreifen kann und im Zuge der Bewegung des zweiten Elements 14 in das Eintrittsloch 33 hinein, die Schenkel 26, 27 in Richtung der Pfeile 34 voneinander weg bewegt, da die Abschrägung 27 für die Klauenschenkel 26, 27 in diesem Falle eine schiefe Ebene bildet. Bei Weiterbewegung über den vorderen zylindrischen Teil des zweiten Elements 14 werden dann, wenn das zweite Element 14 seine konstruktiv bestimmte Endstellung im Eintrittsloch 33 des ersten Elements 12 erreicht hat, auch die Klauenschenkel 26, 27 mit der Nut 19 fluchen, so daß die Klauenschenkel 26, 27 verriegelnd in die Nut 19 einrasten.

Oder es wird als zweite Möglichkeit des Eintritts des zweiten Elements 14 im Eintrittsloch 33 zum Erreichen der verriegelten Endstellung, wie oben beschrieben, das Klauenelement 22 per Hand bzw. Finger in Richtung des Pfeiles 35, vgl. Fig. 2 bewegt, so daß das zweite Element 14 ungestört von den Schenkeln 26, 27 in seine konstruktiv bedingte Endstellung im Eintrittsloch 33 des ersten Elements 12 gelangen kann, wobei dann, wenn die Endstellung erreicht wird, das Klauenelement 22 einfach losgelassen wird, so daß die Klauenschenkel 26, 27 aufgrund ihrer werkstoffbedingten Federkraft mit ihren schiefen Ebenen 262, 271 längs den schiefen Ebenen 125, 126 des ersten Elements 12 zurückgleiten können. Diese verriegelte Endstellung ist beispielsweise in Fig. 2 dargestellt. Dabei greifen die Klauenschenkel 26, 27 mit ihren kreisabschnittförmigen Bereichen 263, 273 in die Nut 19 ein und die Elemente 12, 14 sind, wie gesagt, miteinander lösbar, vgl. oben, verriegelt.

Die Klauenschenkel 26, 27 können geringfügig aus dem das erste Element 12 bildenden Körper herausstehen, vgl. Fig. 1. Am freien Ende 180 jedes Klauenschenkels 26, 27 ist eine Handhabe 180 vorgesehen, wobei auch die Handhabe 180, die hier in Form einer Verbreiterung der Klauenschenkel 26, 27 ausgebildet ist, im ersten Element 12 in Form einer dort ebenfalls ausgebildeten Führung 210 geführt werden kann. Die Handhabe 181 weist an ihrer Betätigungsseite griffverbessernde Mittel 183 auf, beispielsweise in Form von Rillungen bzw. Vertiefungen, oder es ist dort ein rutschfestes Element vorgesehen.

Auch das erste Element 12, vgl. Fig. 1, kann entsprechende griffverbessernde Mittel 121 an den Seiten 120 aufweisen, die auf die gleiche Weise, wie vorangehend beschrieben, geeignet ausgebildet sein können.

Die Seite 122 des ersten Elements 12, vgl. Fig. 2, die auf der Haut eines Benutzers zu liegen kommt, ist im wesentlichen flächig ausgebildet und kann zudem ergonomisch geformt sein, so daß sie sich im betreffenden Applizierungsbereich der Infusionsvorrichtung 10 am Körper des Benutzers anschmiegen kann, ohne negative Empfindungen seitens des Benutzers während des Tragens der Infusionsvorrichtung 10 hervorzurufen.

Das zweite Element 14 weist an seinem freien Ende 24 eine im wesentlichen axiale Vertiefung 272 auf, in die die Nadel 15 mit ihrem Spitzenbereich 150 hineinsteht. Der in die Vertiefung 172 hineinstehende Spitzenbereich 150 ist derart bemessen, daß die Nadel 15 mit ihrer unmittelbaren Spitze nicht über das freie Ende 24 hinaussteht, vgl. Fig. 9. Aus Fig. 10 ist ersichtlich, daß der Öffnungsbereich 130 der Kanüle 13, der zum freien Ende 24 des zweiten Elements 14 gerichtet ist, derart in das Eintrittsloch 33 hineinsteht, daß im verriegelten Zustand des ersten und des zweiten Elementes 12, 13 die Nadel 15 in ein vor dem Eintrittsloch 33 in der sich dort verbreiternde Öffnung der Kanüle 13 angeordnetes Verschlußelement 131 eindringen kann. Die Spitze der Nadel 15 kommt dann im verbreiterten Öffnungsbereich der Kanüle 13, vgl. Fig. 6, zu liegen. Das Verschlußelement 131, das mit Führungsmitteln 133, vgl. Fig. 10, an seinem bestimmungsgemäßen Ort vor dem in Richtung des Eintrittsloches 33 sich vergrößernden Öffnungsbereich 130 sitzt, kann aus elastomerem und/oder polymerem Werkstoff ausgebildet sein, beispielsweise aus Gummi oder einem anderen geeigneten human- bzw. tiermedizinisch unbedenklichen Werkstoff.

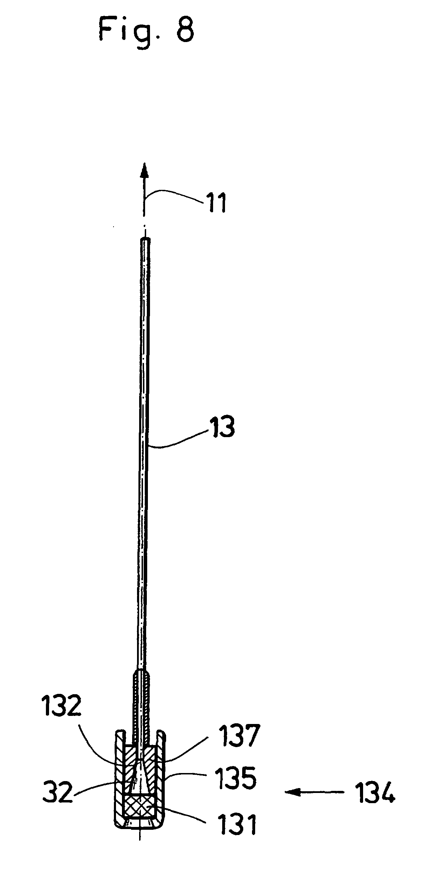

Die Kanüle 13 ist an ihrem im ersten Element 12 zu liegen kommenden Kanülenende 134, vergleiche Fig. 8, mit einem Hülsenelement 135 umgeben. Im Hülsenelement 135 ist das Verschlußelement 131 angeordnet und in Richtung des austrittseitigen Endes des Therapeutikums 11 aus der Kanüle, d.h. vor dem Verschlußelement 131, ist ein Verbindungselement 137 angeordnet, über das die Kanüle 13 mit dem Hülsenelement 135 verbunden ist. Die Kanüle 13 sowie das Hülsenelement 135 und das darin aufgenommene Verschlußelement 131 sowie das Verbindungselement 137 bilden eine Montageeinheit, die in das erste Element 12 in die dort im Inneren ausgebildete sackartig ausgebildete Öffnung 127 eingesetzt werden kann. Die Öffnung 127 ist vorzugsweise als Preßsitz für das Hülsenelement 135 ausgebildet, so daß keine weiteren Halt- bzw. Sicherungsmaßnahmen zwischen dem ersten Element 12 und dem Hülsenelement 135 vorgesehen werden müssen.

Zwischen der Achse der Kanüle 13 und der im wesentlichen flächig ausgebildeten Unterseite 122 des ersten Elements 12 kann ein stumpfer Winkel im Bereich von 183° ausgebildet sein, so daß die Kanüle 13 relativ zum Körper des ersten Elements 12 geneigt ist.

Es wird schließlich Bezug genommen auf die Darstellung der Infusionseinrichtung 10 gemäß Fig. 7. Das dort dargestellte zweite Element 140 unterscheidet sich von dem in den übrigen Figuren dargestellten zweiten Element 14 dadurch, daß das zweite Element 140 eine sehr viel längere Nadel 151 als die Nadel 15 aufweist. Das zweite Element 140 gemäß Fig. 7 ist nicht mit einem Schlauchelement 17 für die Zufuhr eines Therapeutikums 11 verbunden, vielmehr ist dort die Nadel 151 auf geeignete Weise im vorderen Bereich des zweiten Elements 140, d.h. dem Bereich, mit dem das zweite Element 140 auf ansonsten gleiche Weise, wie vorangehend beschrieben, verbunden und wird mit dem ersten Element 12 genauso verbunden wie das zweite Element 14. Die Nadel 151 ist derart lang, daß diese im mit ihrem Spitzenbereich 150 verrasteten Zustand des ersten und zweiten Elements 12, 140 aus der normalerweise zum Austritt des zu verabreichenden Therapeutikums 11 bestimmten Spitze der Kanüle geringfügig heraussteht, vergleiche Fig. 7.

Wenn die Infusionseinrichtung 10 erstmals verwendet wird, wird die Konfiguration gemäß Fig. 7 verwendet. Die Nadel 151 kann auf bekannte Weise leicht in das Körperinnere des zu therapierenden Menschen oder Tieres eingeführt werden, wobei im Zuge des Einführens der Nadel 151 ebenfalls die Kanüle 13 leicht in das Körperinnere eingeführt werden kann. Beim Erreichen der vorbestimmten Tiefe im Körperinneren wird auf oben beschriebene Weise das zweite Element 141 aus dem ersten Element 12 herausgezogen und anstelle des zweiten Elements 140 das zweite Element 14 eingesetzt, und zwar derart, wie es in der in Fig. 6 dargestellten Endstellung gezeigt ist. Dabei durchstößt die mit einem Hohlraum versehene Nadel 15 das Verschlußelement 131, so daß über das Schlauchelement 17 durch die Nadel 15 zugeführtes Therapeutikum 11 in die Kanüle 13 und von dort in das Körperinnere des zu therapierenden Menschen bzw. Tieres gelangen kann. Wird zum Abschluß des Thearpiervorganges bei im Körperinneren positionierter Kanüle 13 das erste Element 14 entfernt, verschließt sich das Verschlußelement 131 schmutz-, flüssigkeits- und keimdicht, nachdem die Nadel 15 zusammen mit dem zweiten Element 14 aus dem ersten Element 12 herausgezogen worden ist.

Die in Verbindung mit dem modifizierten zweiten Element 140 verwendete Einführnadel 151 kann hohlraumfrei sein, sie kann aber auch nach Art einer Injektionsnadel mit einem axial durchgehenden Hohlraum versehen sein. Ist die lange Einführnadel 151 mit einem durchgehenden Hohlraum versehen, wird bei dieser eine vom äußeren der Nadel aus in den Hohlraum hineinführende Öffnung 136 vorgesehen sein, vergleiche Fig. 7. Die Öffnung 136 ist derart vorgesehen, daß im zusammengeführten Zustand des ersten und des zweiten Elements 12, 140 diese im Bereich der elementseitigen 12 Öffnung der Kanüle 13 vorgesehen ist, d.h. bei der Ausgestaltung der Infusionseinrichtung 10 gemäß Fig. 7 mündet die Öffnung 136 in die sich zum zweiten Element 140 verbreiternde Öffnung bzw. den Hohlraum im Verbindungselement 137, das im Hülsenelement 135 angeordnet ist. Diese Ausgestaltung der Einführnadel 151 mit durchgehendem Hohlraum und Öffnung 136 wird dann vorgesehen, wenn die Infusionseinrichtung 10 im Zuge der Sterilisierung nicht mit ionisierenden Strahlen, beispielsweise β-Strahlen, sterilisiert wird, sondern mit einem ggf. stark oxidierenden Gas. Das Gas kann dann vom Spitzenbereich 150 der Einführnadel 151 durch den Hohlraum der Einführnadel 151 bis in den Bereich der elementseitigen 12 Kanülenöffnung 132 gelangen und von dort aus über die Öffnung 136 in den sich trichterförmig erweiternden Innenraum des Verbindungselements 137. Von dort kann bei geeignetem Druck des oxidierenden Gases das Gas zwischen dem zwangsweise gebildeten ringförmigen Innenraum zwischen der Einführnadel 151 und der Kanüle 13 wieder zum austrittseitigen Ende der Kanüle 13 geführt werden und ggf. von dort zurück in den Kreislauf des Sterilisierungsgases.

### Bezugszeichenliste

- 10: Infusionseinrichtung
- 11: Therapeutikum
- 12: erstes Element
- 120: Seite
- 121: griffverbesserndes Mittel
- 122: Seite
- 123: Rastvorsprung
- 124: Rastvorsprung
- 125: schiefe Ebene
- 126: schiefe Ebene
- 127: Öffnung
- 13: Kanüle
- 130: Öffnungsbereich
- 131: Verschlußelement
- 132: Kanülenöffnung
- 133: Führungsmittel
- 134: Kanülenende
- 135: Hülsenelement
- 136: Öffnung
- 137: Verbindungselement
- 14: zweites Element
- 140: zweites Element
- 15: Nadel
- 150: Spitzenbereich
- 151: Einführnadel
- 16: Hohlraum
- 17: Schlauchelement
- 18 180 181: Handhabe
- 182: Betätigungsseite
- 183: griffverbesserndes Mittel
- 19: Nut
- 20: Achse, axialsymmetrisch (zweites Element)
- 21: Führung
- 210: Führung
- 22: Klauenelement
- 23: Einführabschnitt
- 24: freies Ende (zweites Element)
- 25: Abschrägung
- 26: Klauenschenkel
- 260: freies Ende
- 261: Bereich (schiefe Ebene)
- 262: Rastnase
- 263: kreisabschnittförmiger Bereich
- 27: Klauenschenkel
- 270: freies Ende
- 271: Bereich (schiefe Ebene)
- 272: Rastnase
- 273: kreisabschnittförmiger Bereich
- 28: Radius
- 29 30: Führungsbahn
- 31: Führungsbahn
- 32: Lochachse (erstes Führungselement)
- 33: Eintrittsloch
- 34: Pfeil
- 35: Pfeil

## Patentansprüche

1. Infusionseinrichtung (10) für die Verabreichung eines Therapeutikums (11) in das Körperinnere eines Menschen oder eines Tieres, umfassend wenigstens ein erstes Element (12), an dem eine Kanüle (13) zum Einführen in das Körperinnere und zum Sitz im Körperinneren angeordnet ist, und ein zweites Element (14), das eine Nadel (15) mit einem durchgehenden Hohlraum (16) nach Art einer Injektionsnadel aufweist, die mit einem Schlauchelement (17) für die Zufuhr des Therapeutikums (11) verbunden ist, wobei das erste und das zweite Element (12, 14) über ein Rastelement lösbar derart miteinander verbindbar sind, daß das Therapeutikum (11) vom Schlauchelement (17) über die Nadel (15) in die Kanüle (13) gelangen kann, **dadurch gekennzeichnet, daß** das Rastelement durch eine im wesentlichen kreisringförmig umlaufende Nut (19) im im wesentlichen axialsymmetrisch (20) zur Nadel (15) ausgebildeten zweiten Element (14) und durch wenigstens ein im Verbindungszustand im wesentlichen orthogonal zum zweiten Element (14) im ersten Element (12) geführtes (21) und hin- und herverschiebbares Klauenelement (22), das im Verbindungszustand beider Elemente (12, 14) in die Nut (19) eingreifend beide Elemente (12, 14) miteinander verriegelt hält, ausgebildet wird.

2. Infusionseinrichtung nach Anspruch 1, **dadurch gekennzeichnet, daß** das zweite Element (14) einen im wesentlichen kreisförmigen Querschnitt aufweist, bei dem die Kreisachse bzw. der Kreismittelpunkt mit der Achse (20) der Nadel (15) zusammenfällt.

3. Infusionseinrichtung nach einem oder beiden der Ansprüche 1 oder 2, **dadurch gekennzeichnet, daß** der in das erste Element (12) hineinzuführende Abschnitt (23) des zweiten Elements (14) an seinem freien Ende (24) eine im wesentlichen umlaufende Abschrägung (25) aufweist.

4. Infusionseinrichtung nach einem oder mehreren der Ansprüche 1 bis 3, **dadurch gekennzeichnet, daß** das Klauenelement (22) durch zwei gesonderte Klauenschenkel (26, 27) gebildet wird, die im wesentlichen parallel zueinander geführt aufeinander zu bzw. voneinander weg bewegbar sind.

5. Infusionseinrichtung nach Anspruch 4, **dadurch gekennzeichnet, daß** die Klauenschenkel (26, 27) einen kreisabschnittförmigen Bereich (263, 273) aufweist, mit einem Radius (28) entsprechend dem Radius (28) der Nut (19) des zweiten Elements (14), mit dem er in die Nut (19) verriegelnd eingreift.

6. Infusionseinrichtung nach einem oder beiden der Ansprüche 4 oder 5, **dadurch gekennzeichnet, daß** der Klauenschenkel (26, 27) einen in Form einer schiefen Ebene ausgebildeten Bereich (261, 271) aufweist, wobei in der als Führungsschlitz (21) ausgebildeten Führung im ersten Element (12) ebenfalls als schiefe Ebene ausgebildete, gegensinnig für jeweils einen Klauenschenkel (26, 27) ausgebildete Bereiche (125, 126) vorgesehen sind, wobei zum Entriegeln der beiden Elemente (12, 14) voneinander die als schiefen Ebenen ausgebildeten Bereiche (261, 271) der jeweiligen Klauenschenkel (26, 27) an den jeweils zugeordneten, als schiefe Ebenen ausgebildeten Bereichen (125, 126) des ersten Elements (12) aufgleiten und dabei die Klauenschenkel (26, 27) derart elastisch verformt werden, daß sie außer Eingriff mit der Nut (19) kommen.

7. Infusionseinrichtung nach einem oder mehreren der Ansprüche 1 bis 6, **dadurch gekennzeichnet, daß** die Führung (21) für die Klauenschenkel (26, 27) durch einen im wesentlichen orthogonal zur Achse (32) des im wesentlichen im Querschnitt kreisförmigen Eintrittslochs (33) für das zweite Element (14) verlaufenden Führungsschlitz (210) ausgebildet ist.

8. Infusionseinrichtung nach einem oder mehreren der Ansprüche 4 bis 6, **dadurch gekennzeichnet, daß** die Klauenschenkel (26, 27) eine Rastnase (262, 272) aufweisen, mit der sie hinter Rastvorsprünge (123, 124), die im Bereich der Führung (21) im ersten Element (12) ausgebildet sind, nach Art einer Klippverbindung rastend greifen.

9. Infusionseinrichtung nach Anspruch 8, **dadurch gekennzeichnet, daß** die freien Enden (180) der Klauenschenkel (26, 27) mit einer Handhabe (181) versehen ist.

10. Infusionseinrichtung nach Anspruch 9, **dadurch gekennzeichnet, daß** die Handhabe (181) im ersten Element (12) wenigstens teilweise geführt (210) aufgenommen wird.

11. Infusionseinrichtung nach einem oder beiden der Ansprüche 8 oder 9, **dadurch gekennzeichnet, daß** die Handhabe (181) an ihrer Betätigungsseite (182) ein griffverbesserndes Mittel (183) aufweist.

12. Infusionseinrichtung nach einem oder mehreren der Ansprüche 1 bis 11, **dadurch gekennzeichnet, daß** wenigstens eine Seite (122) des das erste Element (12) bildenden Körpers im wesentlichen flächig ausgebildet ist.

13. Infusionseinrichtung nach einem oder mehreren der Ansprüche 3 bis 12, **dadurch gekennzeichnet, daß** das zweite Element (14) an seinem freien Ende (24) eine im wesentlichen axiale Vertiefung (272) aufweist.

14. Infusionseinrichtung nach Anspruch 13, **dadurch gekennzeichnet, daß** die Nadel (15) mit ihrem Spitzenbereich (150) in die Vertiefung hineinsteht, ohne über das freie Ende (24) hinauszuragen.

15. Infusionseinrichtung nach einem oder beiden der Ansprüche 13 oder 14, **dadurch gekennzeichnet, daß** im miteinander verbundenen und verriegelten Zustand beider Elemente 12, 14 der elementseitige Öffnungsbereich (130) der Kanüle (13) wenigstens teilweise in die Vertiefung (272) hineinragt.

16. Infusionseinrichtung nach Anspruch 15, **dadurch gekennzeichnet, daß** der öffnungsbereich (130) mit einem diesen abschließenden Verschlußelement (131) versehen ist.

17. Infusionseinrichtung nach Anspruch 16, **dadurch gekennzeichnet, daß** das Verschlußelement (131) aus einem elastomeren und/oder plastomeren Werkstoff ausgebildet ist.

18. Infusionseinrichtung nach einem oder mehreren der Ansprüche 1 bis 17, **dadurch gekennzeichnet, daß** wenigstens die beiden Elemente (12, 14) und das Klauenelement (22) aus Kunststoff, insbesondere einem spritzfähigen Kunststoff, hergestellt sind.

19. Infusionseinrichtung nach Anspruch 18, **dadurch gekennzeichnet, daß** das Klauenelement (22) aus Polyacetal POM besteht.

20. Infusionseinrichtung nach einem oder mehreren der Ansprüche 1 bis 19, **dadurch gekennzeichnet, daß** die Kanüle (13) an ihrem im ersten Element (12) zu liegen kommenden Kanülenende (134) mit einem Hülsenelement (135)-umgeben ist, über das die Kanüle (13) in einer am Ende des Eintrittslochs (33) für das zweite Element (14) sacklochartig ausgebildeten Öffnung (127) im ersten Element (12) aufgenommen wird.

21. Infusionseinrichtung nach Anspruch 20, **dadurch gekennzeichnet, daß** die Öffnung (127) einen Sitz, vorzugsweise einen Preßsitz, für das Hülsenelement (135) bildet.

22. Infusionseinrichtung nach einem oder beiden der Ansprüche 20 oder 21, **dadurch gekennzeichnet, daß** das Verschlußelement (131) im Hülsenelement (135) angeordnet ist.

23. Infusionseinrichtung nach Anspruch 22, **dadurch gekennzeichnet, daß** im Hülsenelement (135) in Richtung des austrittsseitigen Endes des Therapeutikums (11) aus der Kanüle (13) vor dem Verschlußelement (131) ein Verbindungselement (137) angeordnet ist, über das die Kanüle (13) mit dem Hülsenelement (135) verbunden ist.

24. Infusionseinrichtung nach einem oder mehreren der Ansprüche 1 bis 23, **dadurch gekennzeichnet, daß** das zweite Element (140) zum erleichterten Einführen der Kanüle (13) in das Körperinnere mit einer derart langen, Nadel (151) versehen ist, daß diese im verrasteten Zustand des ersten und zweiten Elements (12, 140) aus der zum Austritt des zu verabreichenden Therapeutikums bestimmten Spitze der Kanüle (13) heraussteht.

25. Infusionseinrichtung nach Anspruch 24, **dadurch gekennzeichnet, daß** die Nadel (151) nach Art einer Infusionsnadel mit einem axial durch die Nadel (151) hindurchgehenden Hohlraum versehen ist, wobei eine vom äußeren der Nadel aus in den Hohlraum hineinführende Öffnung (136) vorgesehen ist, die im zusammengeführten Zustand des ersten und des zweiten Elements (12, 140) im Bereich der elementseitigen Öffnung (136) der Kanüle (13) vorgesehen ist.

## Claims

1. Infusion device (10) to administer a therapeutic substance (11) into the interior of the body of a human being or of an animal, comprising at least a first element (12) on which is arranged a cannula (13) to be introduced into the interior of the body and to be in position in the interior of the body, and a second element (14) that has a needle (15) with a hollow cavity (16) passing entirely through it like an injection needle, and which is attached to a tube element (17) to supply the therapeutic substance (11), whereby the first and the second element (12, 14) are detachably connectable to one another via a detent element in such a way that the therapeutic substance (11) can pass from the tube element (17) via the needle (15) into the cannula (13), **characterized in that** the detent element consists of a groove (19) running around essentially in an annular shape in the second element (14) whose shape is essentially axially symmetrical (20) relative to the needle (15), and of at least one claw element (22) that can be slid to and fro and is introduced (21) into the first element (12) essentially at right angles to the second element (14) in the connected state and which, when the two elements (12, 14) are in the connected state, interlocks the two elements (12, 14) to one another by engaging in the groove (19).

2. Infusion device according to Claim 1, **characterized in that** the second element (14) has an essentially circular cross-section in which the circle axis or circle centre point coincides with the axis (20) of the needle (15).

3. Infusion device according to one or both of the Claims 1 or 2, **characterized in that** the section (23) of the second element (14) that is to be inserted into the first element (12) has at its free end (24) a bevel (25) running round essentially peripherally.

4. Infusion device according to one or more of the Claims 1 to 3, **characterized in that** the claw element (22) is formed by two separate claw legs (26, 27) that are movable towards one another and away from one another, being guided essentially parallel to each other.

5. Infusion device according to Claim 4, **characterized in that** the claw legs (26, 27) have a region (263, 273) shaped like a circle segment with a radius (28) corresponding to the radius (28) of the groove (19) of the second element (14) with which it engages in an interlocking manner in the groove (19).

6. Infusion device according to one or both of the Claims 4 or 5, **characterized in that** the claw leg (26, 27) has a region (261, 271) in the shape of an inclined plane, whereby regions (125, 126) are provided in the guide in the first element (12) that is constructed as a guide slot (21), again constructed as an inclined plane and in opposite directions each for one claw leg (26, 17), whereby to unlatch the two elements (12, 14) from one another, the regions (261, 271) of the respective claw leg (26, 27) shaped as inclined planes slide on the regions (125, 126) of the first element (12) in the shape of inclined planes and allocated to each of them, and in the process the claw legs (26, 27) are elastically deformed in such a way that they disengage from the groove (19).

7. Infusion device according to one or more of the Claims 1 to 6, **characterized in that** the guide (21) for the claw legs (26, 27) is formed by a guide slot (210) running essentially at right angles to the axis (32) of the essentially circular cross-section entry hole (33) for the second element (14).

8. Infusion device according to one or more of the Claims 4 to 6, **characterized in that** the claw legs (26, 27) have a detent lug (262, 272) with which they engage in a latching manner behind detent projections (123, 124) formed in the region of the guide (21) in the first element (12) like a clip connector.

9. Infusion device according to Claim 8, **characterized in that** the free ends (180) of the claw legs (26, 27) are equipped with a handling device (181).

10. Infusion device according to Claim 9, **characterized in that** the handling device (181) in the first element (12) is accommodated at least partly in a guided manner (210).

11. Infusion device according to one or both of the Claims 8 or 9, **characterized in that** the handling device (181) has means for improving grip (183) on its operating side (182).

12. Infusion device according to one or more of the Claims 1 to 11, **characterized in that** at least one side (122) of the body forming the first element (12) is constructed essentially as an extended flat surface.

13. Infusion device according to one or more of the Claims 3 to 12, **characterized in that** the second element (14) has a an essentially axial depression (272) at its free end (24).

14. Infusion device according to Claim 13, **characterized in that** the tip region (150) of the needle (15) enters the depression without projecting beyond the free end (24).

15. Infusion device according to one or both of the Claims 13 or 14, **characterized in that** the opening region (130) of the cannula (13) on the element side projects at least partly into the depression (272) when the two elements 12, 14 are in the connected and interlocked state.

16. Infusion device according to Claim 15, **characterized in that** the opening region (130) is provided with a closure element (131) that closes it.

17. Infusion device according to Claim 16, **characterized in that** the closure element (131) is constructed from an elastomeric and/or plastomeric construction material.

18. Infusion device, according to one or more of the Claims 1 to 17, **characterized in that** at least the two elements (12, 14) and the claw element (22) are manufactured from plastic, in particular a plastic that can be injection-moulded.

19. Infusion device according to Claim 18, **characterized in that** the claw element (22) consists of polyacetal POM.

20. Infusion device according to one or more of the Claims 1 to 19, **characterized in that** the cannula (13), at its cannula end (134) that ultimately rests in the first element (12), is surrounded by a sleeve element (135) via which the cannula (13) is accommodated in an opening (127) shaped like a blind hole in the first element (12) at the end of the entry hole (33) for the second element (14).

21. Infusion device according to Claim 20, **characterized in that** the opening (127) forms a seat, preferably a press-fit seat, for the sleeve element (135).

22. Infusion device according to one or both of the Claims 20 or 21, **characterized in that** the closure element (131) is arranged in the sleeve element (135).

23. Infusion device according to Claim 22, **characterized in that** there is arranged in the sleeve element (135) in the direction of the end where the therapeutic substance (11) emerges from the cannula (13), upstream of the closure element (131), a connector element (137) via which the cannula (13) is connected to the sleeve element (135).

24. Infusion device according to one or more of the Claims 1 to 23, **characterized in that**, in order to simplify the insertion of the cannula (13) into the interior of the body, the second element (140) is provided with a needle (151) of such a length that, when the first and second elements (12, 140) are in the interlocked state, this (needle) projects from the tip of the cannula (13) intended for the emergence of the therapeutic substance that is to be administered.

25. Infusion device according to Claim 24, **characterized in that** the needle (151) is provided with a hollow cavity passing axially through the needle (151) like an infusion needle, whereby there is provided an opening (136) that leads from the exterior of the needle into the hollow cavity and which, when the first and second elements (12, 140) are in the assembled state, is provided in the region of the opening (136) of the cannula (13) on the element side.

## Revendications

1. Dispositif de perfusion (10). pour l'administration d'un agent thérapeutique (11) à l'intérieur du corps d'un être humain ou d'un animal, comprenant au moins un premier élément (12), dans lequel est prévue une canule (13) pour être insérée à l'intérieur du corps et maintenue à l'intérieur du corps, et un second élément (14) qui présente une aiguille (15), comprenant un espace creux (16) ininterrompu, du type aiguille à injection qui est reliée à un élément tubulaire (17) pour l'acheminement de l'agent thérapeutique (11) le premier et le second élément (12, 14) pouvant être reliés l'un à l'autre de manière amovible à l'aide d'un élément d'encliquetage de façon à ce que l'agent thérapeutique (11) puisse parvenir depuis l'élément tubulaire (17) par l'intermédiaire de l'aiguille (15) jusque dans la canule (13), **caractérisé en ce que** l'élément d'encliquetage est formé par une rainure (19) périphérique essentiellement circulaire dans le second élément (14) réalisé essentiellement de manière axisymétrique (20) par rapport à l'aiguille (15) et par au moins une griffe (22) qui, en position encliquetée, est logée dans le premier élément (12) essentiellement orthogonalement par rapport au second élément (14) et qui est déplaçable selon un mouvement de va-et-vient et qui, en position encliquetée des deux éléments (12, 14), maintient ensemble les deux éléments (12, 14) en position verrouillée en s'engageant dans la rainure (19).

2. Dispositif de perfusion selon la revendication 1, **caractérisé en ce que** le second élément (14) présente une section essentiellement circulaire, dans laquelle l'axe du cercle ou le centre du cercle coïncide avec l'axe (20) de l'aiguille (15).

3. Dispositif de perfusion selon l'une ou les deux des revendications 1 et 2, **caractérisé en ce que** le segment (23) du second élément (14) devant être inséré dans le premier élément (12) présente, au niveau de son extrémité libre (24), un chanfrein (25) essentiellement périphérique.

4. Dispositif de perfusion selon l'une ou plusieurs des revendications 1 à 3, **caractérisé en ce que** la griffe (22) est formée par deux branches de griffe (26, 27) séparées que l'on peut déplacer essentiellement parallèlement l'une en direction de l'autre ou dans la direction opposée.

5. Dispositif de perfusion selon la revendication 4, **caractérisé en ce que** les branches de griffe (26, 27) présentent une zone en forme de segment de cercle (263, 273) avec un rayon (28) correspondant au rayon (28) de la rainure (19) du second élément (14), dans lequel elles s'engagent en s'encliquetant dans la rainure (19).

6. Dispositif de perfusion selon l'une ou les deux des revendications 4 et 5, **caractérisé en ce que** la branche de griffe (26, 27) présente une zone (261, 271) réalisée sous forme de plan incliné, des zones (125, 126), réalisées dans le sens opposé à chacune des branches de griffe (26, 27), également réalisées sous forme de plan incliné, étant prévues dans le guidage réalisé sous forme de fente de guidage (21) dans le premier élément (12), où, pour le déverrouillage des deux éléments (12, 14), les zones (261, 271) réalisées sous forme de plans inclinés des branches de griffe respectives (26, 27) glissent sur les zones correspondantes (125, 126) du premier élément réalisées sous forme de plans inclinés et déforment ainsi de manière élastique les branches de griffe (26, 27) de façon à ce qu'elles se désengagent de la rainure (19).

7. Dispositif de perfusion selon l'une ou plusieurs des revendications 1 à 6, **caractérisé en ce que** le guidage (21) pour les branches de griffe (26, 27) est formé par une fente de guidage (210) essentiellement orthogonale par rapport à l'axe (32) de l'alésage d'entrée (33) à section essentiellement circulaire pour le second élément (14).

8. Dispositif de perfusion selon l'une ou plusieurs des revendications 4 à 6, **caractérisé en ce que** les branches de griffe (26, 27) possèdent un cran (262, 272) à l'aide duquel elles s'engagent par encliquetage derrière des parties en saillie (123, 124), qui sont réalisées dans la zone de guidage (21) dans le premier élément (12), à la manière d'un verrouillage à cran.

9. Dispositif de perfusion selon la revendication 8, **caractérisé en ce que** les extrémités libres (180) des branches de griffe (26, 27) sont pourvues d'un élément de préhension (181).

10. Dispositif de perfusion selon la revendication 9, **caractérisé en ce que** l'élément de préhension (181) est au moins partiellement logé de manière guidée (210) dans le premier élément (12).

11. Dispositif de perfusion selon l'une ou les deux des revendications 8 et 9, **caractérisé en ce que** l'élément de préhension (181) présente, sur son côté de maniement (182), un moyen améliorant la préhension (183).

12. Dispositif de perfusion selon l'une ou plusieurs des revendications 1 à 11, **caractérisé en ce qu'**au moins un côté (122) du corps formant le premier élément (12) est réalisé de manière essentiellement plane.

13. Dispositif de perfusion selon l'une ou plusieurs des revendications 3 à 12, **caractérisé en ce que** le second élément (14) présente, sur son extrémité libre (24), un évidement (272) essentiellement axial.

14. Dispositif de perfusion selon la revendication 13, **caractérisé en ce que** l'aiguille (15) rentre, avec la zone de sa pointe (150), dans l'évidement, sans dépasser de l'extrémité libre (24).

15. Dispositif de perfusion selon l'une ou les deux revendications 13 et 14, **caractérisé en ce qu'**en position encliquetée et verrouillée des deux éléments (12, 14), la zone d'ouverture côté élément (130) de la canule (13) rentre au moins en partie dans l'évidement (272).

16. Dispositif de perfusion selon la revendication 15, **caractérisé en ce que** la zone d'ouverture (130) est pourvue d'un élément obturateur (131).

17. Dispositif de perfusion selon la revendication 16, **caractérisé en ce que** l'élément obturateur (131) est réalisé en une matière élastomère et/ou plastomère.

18. Dispositif de perfusion selon l'une ou plusieurs des revendications 1 à 17, **caractérisé en ce qu'**au moins les deux éléments (12, 14) et la griffe (22) sont réalisés en matière plastique, en particulier en une matière plastique injectable.

19. Dispositif de perfusion selon la revendication 18, **caractérisé en ce que** la griffe (22) est composée de polyacétal (POM).

20. Dispositif de perfusion selon l'une ou plusieurs des revendications 1 à 19, **caractérisé en ce que** la canule (13) est entourée, au niveau de l'extrémité de la canule (134) située dans le premier élément (12), par un manchon (135), par l'intermédiaire duquel la canule (13) est logée dans une ouverture (127) de type trou borgne dans le premier élément (12) au niveau de l'extrémité de l'alésage d'entrée (33) pour le second élément (14).

21. Dispositif de perfusion selon la revendication 20, **caractérisé en ce que** l'ouverture (127) forme un logement, de préférence un ajustage serré, pour le manchon (135).

22. Dispositif de perfusion selon l'une ou les deux des revendications 20 et 21, **caractérisé en ce que** l'élément obturateur (131) est disposé dans le manchon (135).

23. Dispositif de perfusion selon la revendication 22, **caractérisé en ce qu'**un élément de liaison (137), qui relie la canule (13) au manchon (135), est disposé dans le manchon (135) dans la direction de l'extrémité de sortie de l'agent thérapeutique (11) hors de la canule (13) et devant l'élément obturateur (131).

24. Dispositif de perfusion selon l'une ou plusieurs des revendications 1 à 23, **caractérisé en ce que** le second élément (140) est pourvu, afin de faciliter l'insertion de la canule (13) à l'intérieur du corps, d'une aiguille (151) possédant une longueur telle qu'elle dépasse, en position encliquetée des premier et second éléments (12, 140), de la pointe de la canule (13) destinée à la sortie de l'agent thérapeutique à administrer.

25. Dispositif de perfusion selon la revendication 24, **caractérisé en ce que** l'aiguille (151) est pourvue, à la manière d'une aiguille à perfusion, d'un espace creux traversant l'aiguille (151) dans le sens axial, une ouverture (136) menant de l'extérieur de l'aiguille à l'espace creux étant prévue dans la zone de l'ouverture (136) côté élément de la canule (13) en position assemblée des premier et second éléments.
